# EUROPEAN PATENT SPECIFICATION

(11) **EP 4 555 980 B1**
(45) Date of publication and mention of the grant of the patent: **21.10.2026**
(21) Application number: 25168432.0
(22) Date of filing: 31.01.2020
(51) Int. Cl.: A61F 5/37, A61F 5/04

(54) **WRIST TRACTION TOWER**
TRAKTIONSTURM FÜR HANDGELENK
TOUR DE TRACTION DE POIGNET

(30) Priority: 31.01.2019 US 201962799318 P; 04.11.2019 US 201962930115 P
(43) Date of publication of application: 21.05.2025
(62) Document number(s) of the earlier application(s) in accordance with Art. 76 EPC: 20708977.2 / 3 917 464
(73) Proprietor: Conmed Corporation, Largo, FL 33773 (US)
(72) Inventor: ALFONSO, Gregory, Seffner, FL, 33584 (US); THIBODEAU, Robert, Saint Petersburg, FL, 33710 (US); SUMMITT, Matthew, Palm Harbor, FL, 34683 (US); VU, Thien, Pinellas Park, FL, 33782 (US); YANTZER, Brenda, Temple Terrace, FL, 33617 (US)
(74) Representative: Strehl & Partner mbB

(56) References cited:
- US-A- 5 419 756
- US-A1- 2005 240 136
- US-B2- 7 857 780

## Description

### CROss-REFERENCE TO RELATED APPLICATION

The present application claims priority to and the benefit of U.S. Provisional Patent Application Number 62/799318, filed on January 31, 2019 and entitled "Wrist Traction Tower," and U.S. Provisional Patent Application Number 62/930115, filed on November 4, 2019 and entitled "Traction Tower Scale".

### BACKGROUND OF THE INVENTION

### 1. Field of the Invention

The present invention relates to orthopedic medical procedure positioning devices/systems and, more particularly, to a wrist traction tower and related traction tower scale.

### 2. Description of Related Art

During arthroscopic surgery in the wrist, for example, surgeons use traction to create enough space in the wrist joint for the appropriate and efficient use of an arthroscope and other related instruments. Conventional traction towers are commonly used to create such traction needed for wrist arthroscopic surgical procedures, radiographic procedures and other related medical procedures. However, conventional traction towers are limited in their ability to accommodate a wide variety of individual patient sizes.

Therefore, there is a need for a traction tower structural design that allows for the adjustability and flexibility to be appropriately sized for each individual patient.

US 2005/240136 A1 and US 7 857 780 B2 disclose orthopedic traction systems allowing for height adjustment for different forearm sizes and rotation of an upper arm portion about a rotation axis perpendicular to an extension axis of the middle portion of the arm.

### BRIEF SUMMARY OF THE INVENTION

Embodiments of the present invention are directed to a wrist traction tower and related traction tower scale. Embodiments of the wrist traction tower are directed to a system with multiple parts, one or more of which are configured, attached, positioned and/or structured to move (e.g., slide, telescope, rotate, twist, turn) with respect to one or more of the other parts of the system. Such adjustability, maneuverability and flexibility provide for an improved and enhanced orthopedic medical procedure positioning system (as compared to conventional devices/systems), which can accommodate a wide variety of lengths and sizes of patients' arms while at the same time providing sufficient space for medical practitioners and their respective equipment to perform surgical, radiographic and other related medical procedures. Elements of the traction tower system of an embodiment can be made of Aluminum, Stainless Steel, Brass, and Plastic (PEEK).

The traction tower assembly of the present invention is defined in Claim 1. Further advantageous features are set out in the dependent claim.

Embodiments of the present invention take into consideration that, in one preferred embodiment, when positioning the height of upper tower and connected arm assembly with respect to the lower tower, an individual patient's wrist joint should be about 2,54cm (1 inch) above the rotation joint (as identified below). This allows a medical practitioner to, for example, x-ray the wrist while keeping it attached to the traction tower. If there is metal too close to the wrist joint, the x-ray image could be affected.

These and other aspects of the invention will be apparent from and elucidated with reference to the embodiment(s) described hereinafter.

### BRIEF DESCRIPTION OF THE SEVERAL VIEWS OF THE DRAWING(S)

The present invention will be more fully understood and appreciated by reading the following Detailed Description in conjunction with the accompanying drawings. The accompanying drawings illustrate only typical embodiments of the disclosed subject matter and are therefore not to be considered limiting of its scope, for the disclosed subject matter may admit to other equally effective embodiments. Reference is now made briefly to the accompanying drawings, in which:
FIG. 1 is an exploded perspective view schematic representation of a traction tower, according to an embodiment.
FIG. 2 is an assembled perspective view schematic representation of the traction tower shown in FIG. 1, according to an embodiment.
FIG. 3 is an assembled perspective view schematic representation of the traction tower shown in FIG. 1, according to an embodiment.
FIG. 4 is a close up perspective view schematic representation of a lower portion of the traction tower shown in FIG. 1, according to an embodiment.
FIG. 5 is a close up perspective view schematic representation of a lower portion of the traction tower shown in FIG. 1, according to an embodiment.
FIG. 6 is an assembled perspective view schematic representation of the traction tower shown in FIG. 1, according to an embodiment.
FIG. 7 is a top view schematic representation of the traction tower shown in FIG. 1, according to an embodiment.
FIG. 8 is a perspective view schematic representation of the traction tower shown in FIG. 1, according to an embodiment.
FIG. 9 is a close up partially sectioned perspective view of a lower portion of the traction tower shown in FIG. 1, according to an embodiment.
FIG. 10 is a perspective view schematic representation of the traction tower shown in FIG. 1, according to an embodiment.
FIG. 11 is a perspective view schematic representation of the traction tower shown in FIG. 1, according to an embodiment.
FIG. 12 is a perspective view schematic representation of the traction tower shown in FIG. 1, according to an embodiment.
FIG. 13 is a perspective view photographic representation of the traction tower with a traction tower scale, according to an embodiment.
FIG. 14A is a perspective view schematic representation of a traction tower scale, according to an embodiment.
FIG. 14B is a perspective view schematic representation of the traction tower scale shown in FIG. 14A, according to an embodiment.
FIG. 15 is an exploded perspective view schematic representation of a traction tower, according to an alternative embodiment.
FIG. 16 is an assembled perspective view schematic representation of the traction tower shown in FIG. 15, according to an alternative embodiment.
FIG. 17 is an assembled perspective view schematic representation of the traction tower shown in FIG. 15, according to an alternative embodiment.
FIG. 18A is a close up partially sectioned perspective view of a lower portion of the traction tower shown in FIG. 15, according to an alternative embodiment.
FIG. 18B is a close up transparent perspective view of a lower portion of the traction tower shown in FIG. 15, according to an alternative embodiment.
FIG. 18C is a close up solid view of a lower portion of the traction tower shown in FIG. 15, according to an alternative embodiment.
FIG. 18D is a close up partially sectioned perspective view of a lower portion of the traction tower shown in FIG. 15, according to an alternative embodiment.
FIG. 18E is a close up bottom perspective view of the rotation joint of the traction tower shown in FIG. 15, according to an alternative embodiment.
FIG. 19 is a close up perspective view schematic representation of a lower portion of the traction tower shown in FIG. 15, according to an alternative embodiment.
FIG. 20 is a close up perspective view schematic representation of a lower portion of the traction tower shown in FIG. 15, according to an alternative embodiment.
FIG. 21 is a perspective view schematic representation of the traction tower shown in FIG. 15, according to an alternative embodiment.
FIG. 22 is a perspective view schematic representation of the traction tower shown in FIG. 15, according to an alternative embodiment.
FIG. 23 is a top view schematic representation of the traction tower shown in FIG. 15, according to an alternative embodiment.
FIG. 24 is a close up partially sectioned perspective view of a lower portion of the traction tower shown in FIG. 15, according to an embodiment.
FIG. 25 is a perspective view schematic representation of the traction tower shown in FIG. 15, according to an alternative embodiment.
FIG. 26 is a is a perspective view schematic representation of the traction tower shown in FIG. 15, according to an alternative embodiment.
FIG. 27 is a perspective view photographic representation of the traction tower, according to an alternative embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the underlying inventive concepts will be apparent to those skilled in the art from this disclosure.

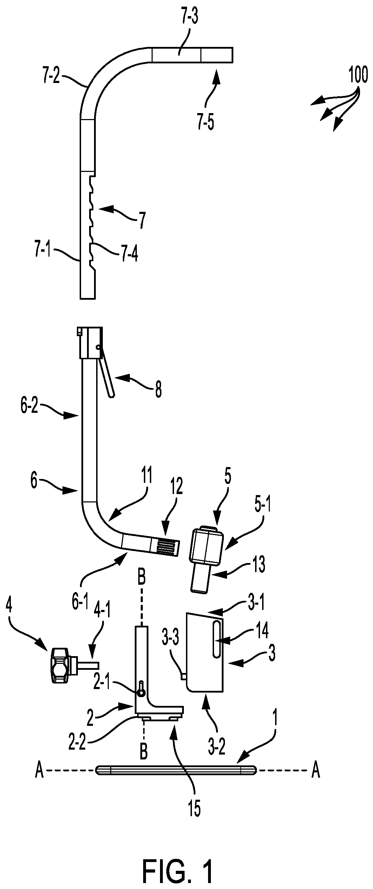

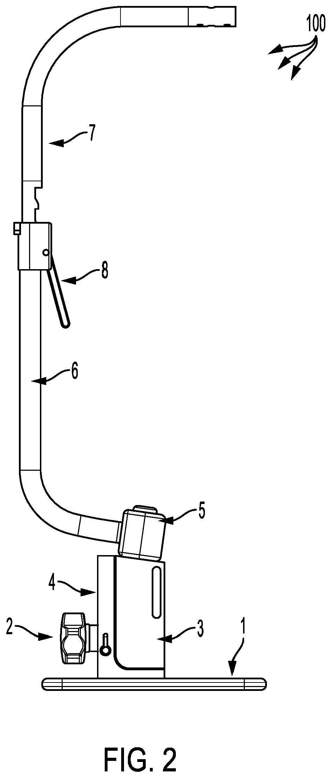

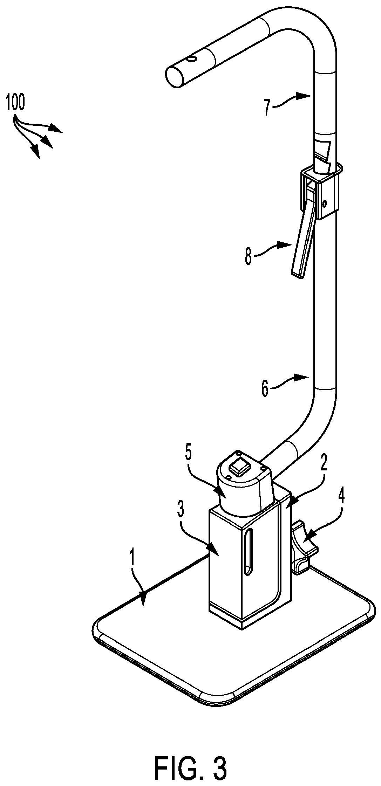

Referring now to the figures, wherein like reference numerals refer to like parts throughout, FIG. 1 shows an exploded perspective view schematic representation of a traction tower 100 according to an embodiment. FIGS. 2 and 3 are assembled perspective view schematic representations of the traction tower 100 shown in FIG. 1, according to an embodiment. As shown, the traction tower 100 includes a base plate 1, a tower assembly, and an arm assembly. The tower assembly is shown having multiple parts - a lower tower 2 and an upper tower 3 - that fit together, and where the upper tower 3 is moveable with respect to the lower tower 2 (as discussed further below). The lower tower 2 is removably securable on the base plate 1 (via any attachment means 15 including a clip, keyed slide and lock mechanism, nut and bolt etc., as should be understood by a person of ordinary skill in the art in conjunction with this disclosure). In addition, the lower tower 2 can include a peg 2-2, which is biased in the downward (protruding from the bottom surface of the lower tower 2) direction via a spring (not shown) positioned in the lower tower 2. The peg 2-2 can be lifted up by sliding button 2-1, and be fully positioned within the lower tower 2. The peg 2-2 can fit into a hole formed in the base plate 1 (not shown), and the lower tower can then be turned (clockwise or counterclockwise) to assist with the locking of the lower tower 2 to the base plate 1. According to an embodiment, all other elements/parts of the traction tower 100 can be, but do not have to be, moveable with respect to at least one other element/part of the traction tower 100.

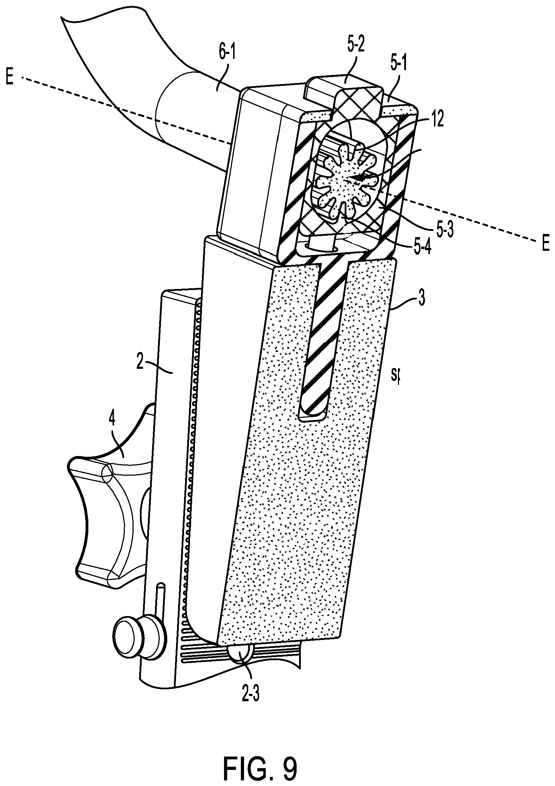

Still referring to FIGS. 1-3, lower tower 2 is L-shaped and is configured to snuggly fit upper tower 3 as shown. The tower assembly can include additional pieces, and can include multiple shapes as long as the pieces fit together in a snug arrangement and the overall movement and locking functionality is similar or remains the same as described herein. The tower assembly components (here the lower tower 2 and upper tower 3), can be secured/locked together by a locking knob 4. The stem 4-1 of locking knob 4 is positionable through lateral apertures positioned in each of lower tower 2 and upper tower 3, and the knob end can be turned to secure each tower component together (as should be understood by a person of skill in the art in conjunction with a review of this disclosure). The aperture in the upper tower 3 is a hole (not shown) shaped to snugly fit, engage and secure the stem of the locking knob 4 when the knob end is turned in the appropriate direction (and be disengaged and be released from the aperture in the upper tower when the knob is turned in the opposite direction). The aperture 2-3 in lower tower 2 is elongated up and down (partially shown in FIG. 9) to allow for upward and downward movement of the upper tower 3 with respect to the lower tower 2, where the upper tower 3 can be resecured to the lower tower 2 per use of the locking knob 4 (as described herein and as should be understood by a person of ordinary skill in the art in conjunction with a review of this disclosure). In accordance with an additional embodiment, the lower tower 2 can include a different or additional elongated aperture(s) to allow for relative movement of the upper tower 3 with respect to the lower tower 2 in a diagonal direction, horizontal direction, or other directions at an angle to the vertical direction B---B when assembled. In accordance with an alternative embodiment, the types of apertures could be reversed between the upper 3 and lower 2 towers. Upper tower 3 also can include an aperture or through hole 14 (longitudinally shaped or other shapes) that can accommodate a strap to hold a patient's forearm to the traction tower 100. The upper tower 3 also includes an alignment peg 3-3, which is configured to fit into a corresponding elongated hole (up and down, not shown) in the lower tower 2. This alignment peg 3-3 acts in conjunction with the stem 4-1 to assist with the alignment of the upper tower 3 with respect to the lower tower 2 and to prevent the upper tower 3 from unwanted movement/rotation with respect to the lower tower 2 when the relative height of the upper tower 3 is adjusted with respect to the lower tower 2.

Continuing to refer to FIGS. 1-3, a rotation joint 5 connects the tower assembly to the arm assembly. A stem 13 of the rotation joint 5 is positionable and rotatable in the upper tower 3 (as shown, positioned through the top surface 3-1 of the upper tower 3, and as discussed further below). The top surface 3-1 of the upper tower is configured to extend along a plane at an angle to the bottom surface 3-2 and/or to the plane of the base plate 1 A---A when assembled. The first end of the lower arm 6 includes a slotted base end 12, which is removably positionable, rotatable and lockable within the head 5-1 of the rotation joint 5 (as discussed further below). Lower arm 6 extends away from the slotted base end 12 and an elongated lower end 6-1 to a curved portion 11 of the lower arm 6, which extends to an elongated upper end 6-2 of the lower arm 6. Upper end 6-2 of the lower arm 6 extends at an angle to an axis (which can be any angle including 45 degrees, substantially perpendicular to perpendicular) of the elongated lower end 6-1. An elongated lower end 7-1 of the upper arm 7, can but does not have to be solid, fits and is telescopically moveable within the elongated upper end 6-2 of the lower arm 6, which is formed as a tube. Lever 8 is connected to elongated upper end 6-2 of the lower arm 6. Lever 8 includes a protrusion or tooth on the end positioned within the elongated upper end 6-2 of the lower arm 6, which can be positioned and fit in between the ridges 7-4 formed on at least one side of the elongated lower end 7-1 of the upper arm 7 (which faces the protrusion or tooth of lever 8 when the elongated lower end 7-1 of the upper arm 7 is positioned within the elongated upper end 6-2 of the lower arm 6). When lever 8 is actuated in a first direction positioning the protrusion or tooth in between one of the pairs of ridges 7-4, the upper arm 7 is fixed/secured/locked with respect to the lower arm 6. When lever 8 is actuated in a second direction, the upper arm 7 is released from its fixed/secured/locked position and is free to move with respect to the lower arm 6 (e.g., further within or without the lower arm 6). In accordance with an alternative embodiment, upper arm 7 can be tubular in structure, and lower arm 6 can be solid/non-tubular (but does not have to be) and contain ridges (essentially the opposite configuration shown in FIG. 1). The lever arm 8 can be any type of actuator including a linear slider, circular actuator or switch etc. (as should be understood by a person of ordinary skill in the art in conjunction with a review of this disclosure).

Elongated lower end 7-1 of the upper arm 7 extends away from the lower arm to a curved portion 7-2 of the upper arm 7, which extends to an elongated upper end 7-3 of the upper arm 7. Upper end 7-3 of the upper arm 7 extends at an angle to an axis (which can be any angle including 45 degrees, substantially perpendicular to perpendicular) of the elongated lower end 7-1, and in essentially the same direction as the elongated lower end 6-1 of the lower arm 6 (and can extend, but does not have to, in a plane that is parallel or substantially parallel thereto; as shown, the elongated lower end 6-1 of the lower arm 6 points slightly more in the relatively downward direction as compared to elongated upper end 7-3 of the upper arm 7, which is shown extending in a plane that is parallel or substantially parallel to plane A---A). Elongated upper end 7-3 of the upper arm 7 includes a through hole 7-5 configured to assist with securing a traction tower scale (embodiments of the traction tower scale and its attachment to a traction tower are discussed further below) thereto.

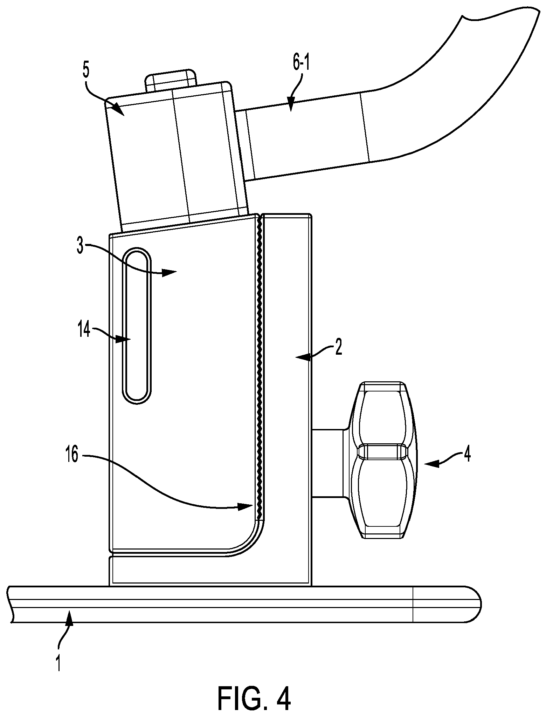

As discussed above, there are several structural features and configurations that allow the traction tower 100 as a whole to be sized appropriately for an individual patient. In addition, as the height of the upper tower 3 is adjusted (as described with respect to FIGS. 4-5 below), the forearm strap position 14 will move with it and is always relatively close to the patient's wrist (the closer to the wrist the better control the strap has). If the strap was in one fixed position it wouldn't work great for different patient sizes.

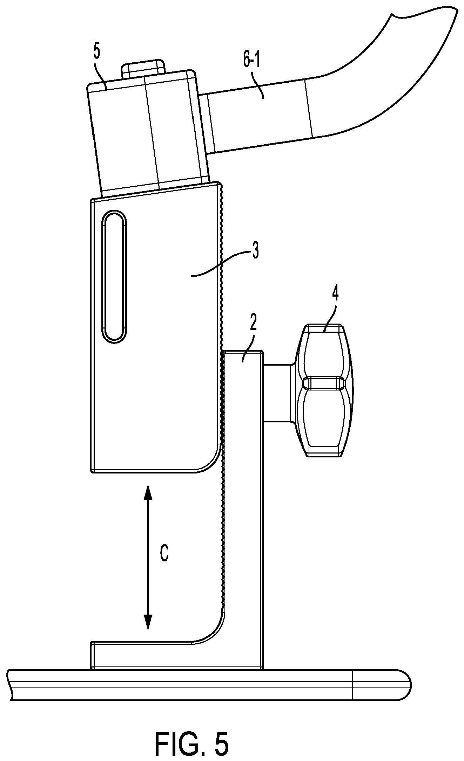

Turning to FIGS. 4-5, close up perspective view schematic representations of a lower portion of the traction tower 100 shown in FIG. 1 are provided, according to an embodiment. FIGS. 4-5 are provided to show movement of upper tower 3 with respect to lower tower 2 to accommodate a variety of individual patent forearm sizes, and the structural features that allow for such movement. FIG. 4 shows the upper tower 3 in its relatively lowest position with respect to lower tower 2, and FIG. 5 shows the upper tower 3 in its relatively highest position with respect to the lower tower 2.

Referring to FIG. 4, the upper tower 3 is shown fitting snuggly within the outline of the lower tower 2 in the upper tower's 3 lowest position. As shown, the upper tower 3 and the lower tower 2 are held together by the locking knob 4 (as discussed above). An interlockable wave/serration pattern 16 may also be provided on each respective lateral facing surface of the upper tower 3 and lower tower 2 to assist with the locking of the upper tower 3 and the lower tower 2 together. The wave/serration pattern 16 can cover the whole of each respective lateral facing surface of the upper tower 3 and lower tower 2, or some portion less than the whole of each respective surface. Also, as discussed above, adjustment of the upper tower 3 upwards is accomplished by (1) loosening the locking knob 4 and connection between the upper tower 3 and lower tower 2, (2) movement of the knob 4 within the elongated aperture 2-1 (not shown) and upper tower 3 up from the position shown in FIG. 4 to the position shown in FIG. 5 and then tightening the locking knob 4 and upper tower 3 in the new position, and (3) as a result of the movement of the upper tower 3 in the up direction along arrow C, a number of other elements move in the up direction with respect to the lower tower 2 including the strap for the patient's arm (not shown) positionable through aperture 14, rotation joint 5, and the arm assembly. To move the upper tower 3 in the opposite direction, the same actions can be performed (i.e., starting with loosening of the locking knob 4, movement of the locking knob 4 and the upper tower 3 etc. in the opposite direction etc.).

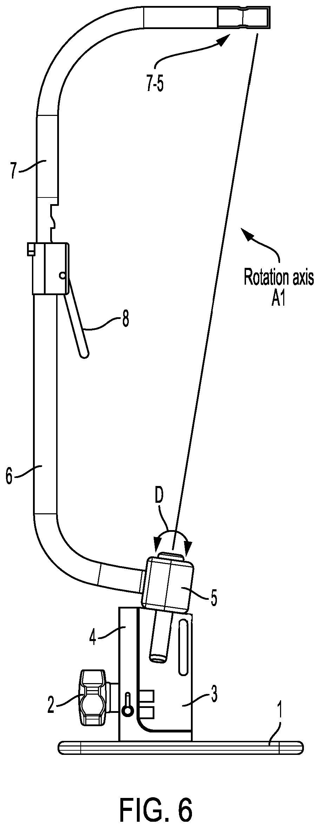

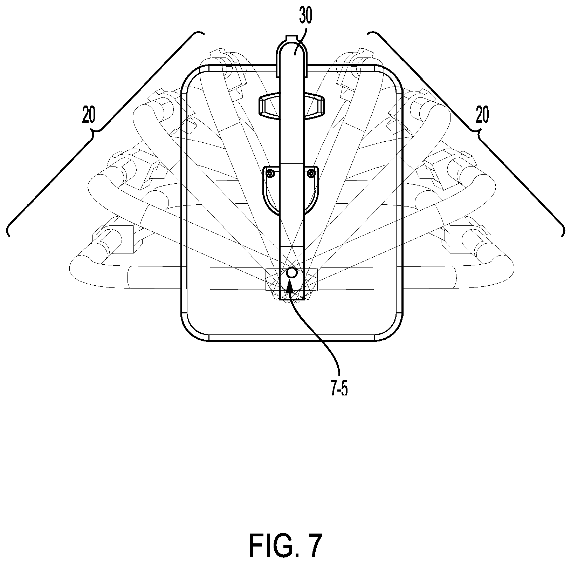

As discussed above, the top surface 3-1 of the upper tower is configured to extend along a plane postitioned at an angle to the plane of the base plate 1 A---A when assembled (see FIG. 1). The rotation joint 5, being attached to the top portion of the upper tower 3, includes a rotation axis A1 extending at an angle to a straight up and down vertical axis - see B---B of FIG. 1. By having the rotation joint 5, which houses a portion of the lower arm 6, positioned at an angle with an angled rotation axis A1 (see, e.g., FIG. 6, which shows the rotation axis A1 created by angling the rotation joint 5 positioned on and its stem 13 positioned within upper tower 3), the rotation of the rotation joint 5 and the arm assembly (explained further below) will not displace the point that the traction is created (as should be understood by a person of ordinary skill in the art in conjunction with a review of this disclosure). This means that the arm assembly can be moved as shown in FIGS. 7-8 without losing traction in the patient's arm or its position.

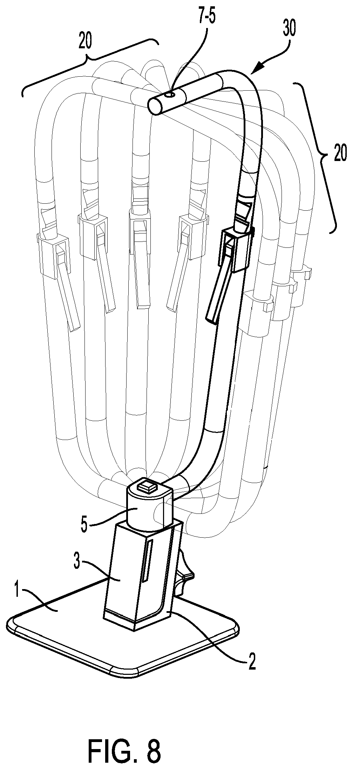

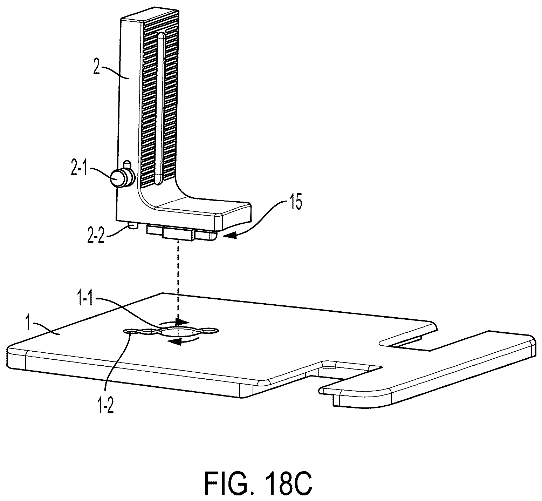

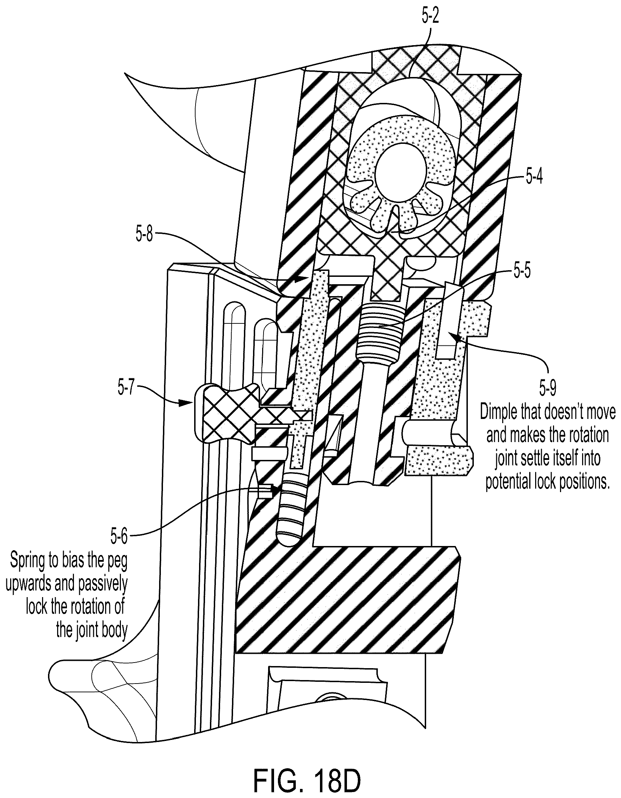

Turning to FIGS. 7 and 8, top and perspective view schematic representations showing the rotation range of rotation joint 5 (and, thus, the arm assembly) of the traction tower 100 about rotation axis A1. The rotation D of the rotation joint 5/arm assembly about the rotation axis A1 (see, e.g., FIG. 6) can be incremental and locked/unlocked via a slotted/tooth embodiment, as shown and described with respect to an additional rotation functionality (see also discussion with respect to FIGS. 18D-E), can be non-incremental when locked/unlocked via frictional engagement, or can be locked/unlocked via other known lock/unlock mechanisms (as should be understood by a person of ordinary skill in the art in conjunction with a review of this disclosure). The rotation range is shown by shadow (transparent) arm assembly structures 20 positioned around the starting or zero position shown by a solid arm assembly structure 30. The position of the screw (not shown) that holds the traction scale at 7-5 remains consistent despite the rotation of the other portions of the arm assembly. As shown in FIG. 8, a large portion of the arm assembly is offset from a patient's arm when in use, regardless of the arm assembly's rotation position, which creates sufficient space for surgical instruments. Stated differently, this structural configuration and associated functionality allows a medical practitioner to move the arm assembly around the patient's hand without affecting the position or traction of the hand itself. The ability to rotate the arm assembly can be important because if the medical practitioner needs more space around the outside of the wrist joint for medical instruments, the medical practitioner can just swing the arm assembly around to the back of the arm. Another use of this structural feature includes allowing the medical practitioner to maneuver the arm assembly in order to get a c-arm (x-ray machine) into position to take an x-ray while the wrist remains in traction.

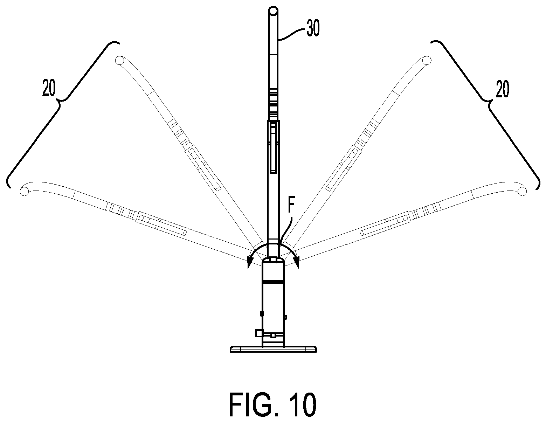

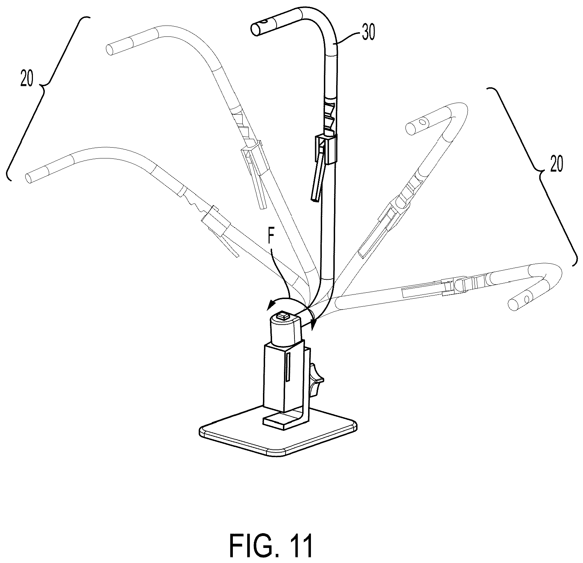

Turning to FIG. 9, a close up partially sectioned perspective view of a lower portion of the traction tower 100 is shown, according to an embodiment. The interface between the slotted base end 12 of the elongated lower end 6-1 of the lower arm 6 and the rotation joint 5 is shown. In particular, the slots/teeth formed on the slotted base end 12 allow for incremental rotation of the arm assembly around a second rotation axis E---E as shown in FIGS. 10 and 11. A main purpose of the rotation of the elongated lower end 6-1 of the lower arm 6 around the second rotation axis E---E is to allow the medical practitioner to control the angle of the patient's wrist while in use by keeping the patient's forearm vertical and pulling traction to the hand at an angle from an elongated axis positioned through the patient's forearm (or vertically), as should be understood by a person of ordinary skill in the art in conjunction with a review of this disclosure. In order to be able to rotate the arm assembly around the second rotation axis E---E, a button 5-2 can be pushed (force exerted against the button 5-2 opposite the spring bias force) to overcome a bias force exerted by a spring (not shown) up against the button housing 5-3 in the direction of the button 5-2 downward to remove a locking tooth 5-4 from being positioned between two respective slots/teeth of the slotted base end 12. When a desired position of the arm assembly is reached, the force exerted against the button 5-2 by the user can be removed and the arm assembly can be locked into the desired position via the described interlocking mechanism (bias force exerted by the spring pushes the locking tooth 5-4 between another two slots/teeth of the slotted base end 12).

Turning to FIGS. 10 and 11, perspective view schematic representations showing the rotation range of the elongated lower end 6-1 of the lower arm 6 (and, thus, the arm assembly) of the traction tower 100 about rotation axis E---E. The rotation F of the elongated lower end 6-1 of the lower arm 6 about the rotation axis E---E can be incremental and locked/unlocked via a slotted/tooth embodiment, as shown and described with respect to FIG. 9), can be non-incremental when locked/unlocked via frictional engagement, or can be locked/unlocked via other known lock/unlock mechanisms (as should be understood by a person of ordinary skill in the art in conjunction with a review of this disclosure). An example of the rotation range for wrist angle control is shown by shadow (transparent) arm assembly structures 20 positioned around the starting or zero position shown by a solid arm assembly structure 30.

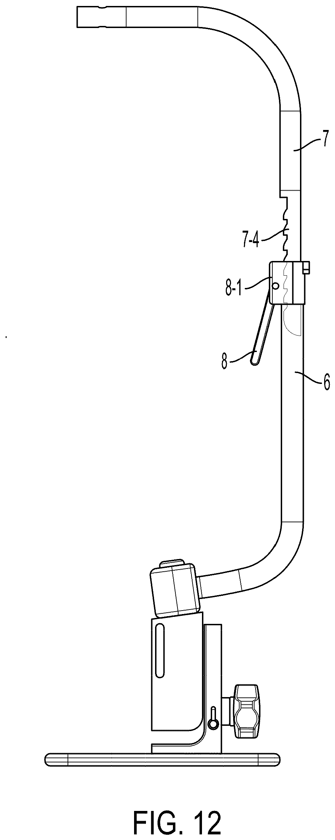

Referring to FIG. 12, a perspective view schematic representation of the traction tower 100 with the lever 8 and ridges 7-4 engagement structure and resulting functionality is shown in a partial transparent view, according to an embodiment. In brief, the height adjustment mechanism is formed between the upper arm 7 and the lower arm 6. This interface between the upper arm 7 and lower arm 6 allows for another adjustment point to be responsive to a wide variety of individual patient arm sizes. The illustrated adjustment mechanism in FIG. 12 includes a ratchet mechanism for quick height adjustment via actuation of lever 8 to position end 8-1 within a selected/particular notch formed in the upper arm 7 at 7-4.

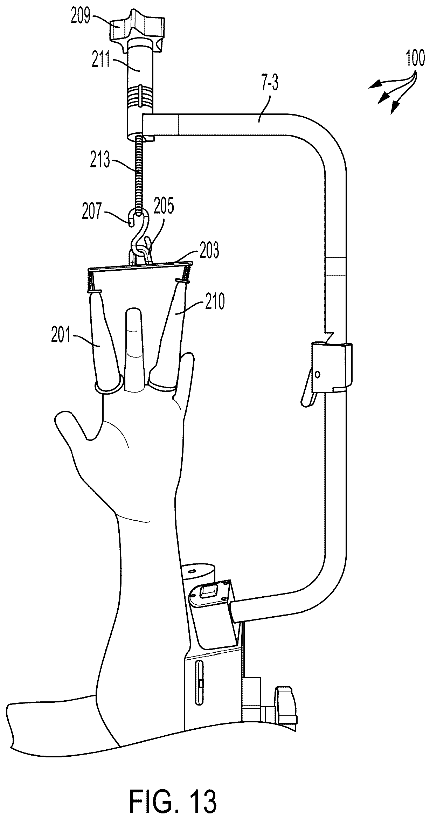

Figures 13-14B illustrate a traction tower scale 200, according to an embodiment. Referring to FIG. 13, a perspective view photographic representation of the traction tower 100 is shown with the traction tower scale 200. The traction tower 200 includes, but is not limited to, a tubular body 211 attached to the distal end of the upper end 7-3 of the upper arm 7 (via welding, screw, nut and bolt or other known attachment means as should be understood by a person of skill in the art in conjunction with a review of this disclosure) of the traction tower 100. The body 211 is configured to contain a knob 209 followed by and attached to a spring (not shown) positioned through the top portion of the body 211. Attached to the bottom end of the spring is a rod/screw 213, which is partially positioned within the body 211 and a portion of which protrudes outside the bottom end of the body 211. The bottom end of the rod/screw 213 includes a hole that receives a clip 207 therethrough. The other end of the clip 207 is attached to a hole formed in or on a rack 203. The rack is shown with two finger traps 201 (but can include one or more than two) attached thereto for securing a patient's fingers and applying traction.

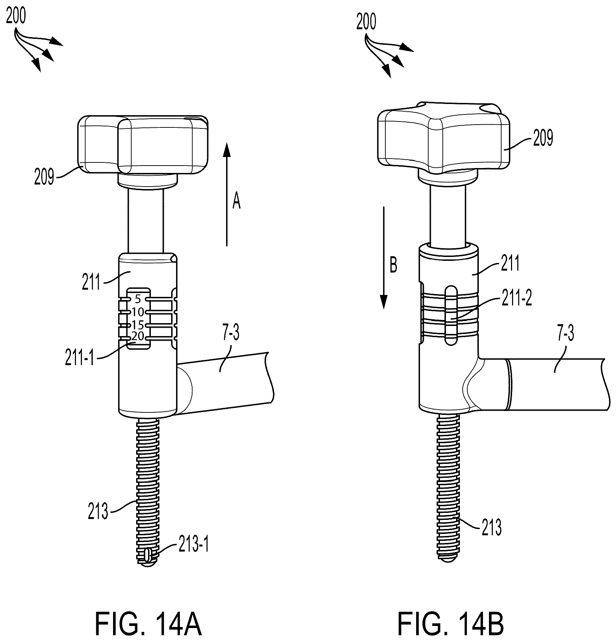

Turning to FIGS. 14A-B, perspective view schematic representations of the traction tower scale 200 are shown, according to an embodiment. The rack 203 (to which the finger traps 201 are attached) is directly linked to the knob 209 through the rod/screw 213 (which can be any type of connection element that can perform the function of the rod/screw 213 described herein, and does not need to be a rod or a screw). In an "at rest" pre-use state (not attached to a patient's fingers, or at least not receiving force from a patient's fingers), the knob 209 is biased in the upward direction (see arrow A) by the spring located in the body (which can be any type of spring including a coil spring with a known biasing force, as should be understood by a person of ordinary skill in the art in conjunction with a review of this disclosure). As a force is applied to the spring in the downward direction (see arrow B) by the weight of a patient's hand/arm (from the finger traps, to the rod/screw 213, to the spring and to the knob 209), the knob 209 (including its stem) will be pulled in the downward direction further into the body 211 of the scale. The stem of the knob 209 can include a visual indicator (e.g., a line or other mark), that can be used to indicate an estimated amount of traction being used. This can be done by viewing where the visual indicator is in the viewing window 211-2, and that location can be matched up with grooves indicating a traction amount number 211-1. The unit of measure for traction is pounds (1 pound = 0,45 kg). However, a preferred embodiment uses the traction amount number as a relative reference traction number (e.g., relative traction amount applied to a patients arm/wrist), and not as a specific measurement device.

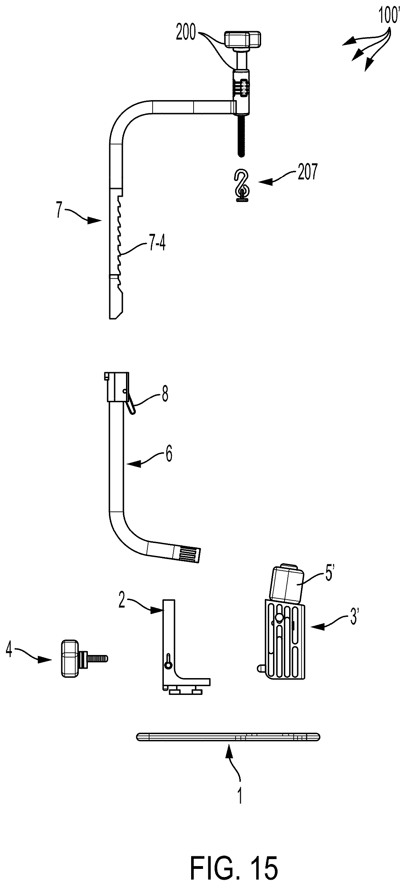

FIGS. 15-26 collectively illustrate a traction tower 100', according to an alternative embodiment. This alternative embodiment of the traction tower 100' is similar in many respects to traction tower 100 (including structurally and functionally), described above with respect to FIGS. 1-12. As such, the following descriptions of the elements/components of the traction tower 100' are mostly limited to the alternative/different aspects (such as upper tower 3' and rotation joint 5'). If a structural element(s) and its resulting (singular or collective) functionality is not discussed but was illustrated and/or discussed above, the structure and associated functionality is the same as what was discussed above with respect to FIGS. 1-12 and applies in this section (similarly, the discussion with respect to the traction tower scale 200 provided in FIGS. 13-14B applies equally below with respect to the traction tower 200 shown as part of the traction tower 100').

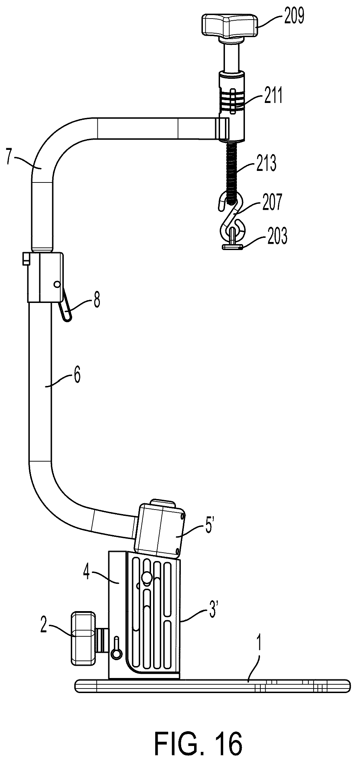

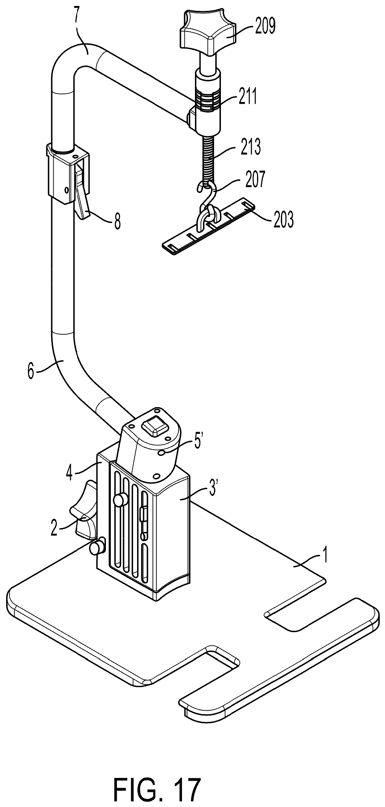

Turning to FIG. 15, an exploded perspective view schematic representation of a traction tower 100' according to an alternative embodiment is shown. FIG. 15 is similar to FIG. 1, except for the addition of the traction tower 200 (described with respect to FIGS. 13-14B) and the alternative embodiment of the upper tower 3' and the rotation joint 5'. FIGS. 16 and 17 are assembled perspective view schematic representations of the traction tower 100' shown in FIG. 15, according to an alternative embodiment, and are similar to FIGS. 2 and 3, respectively.

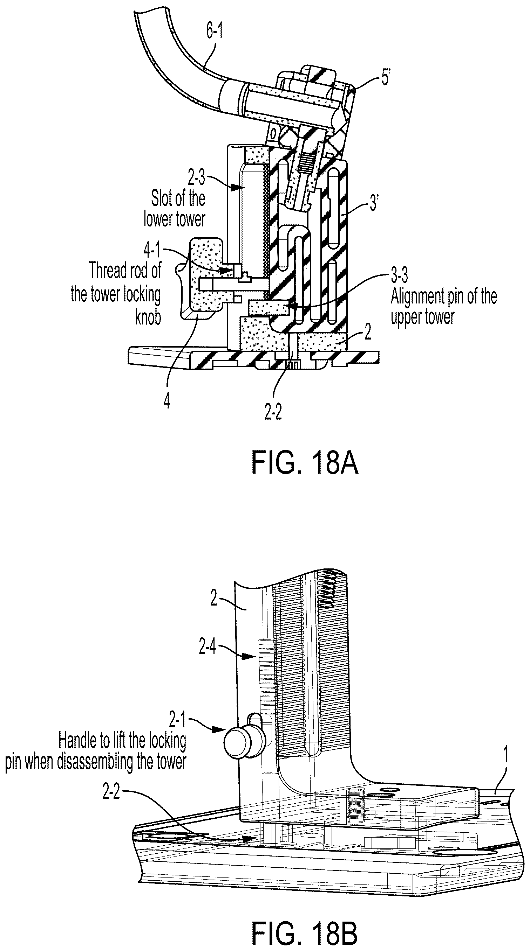

FIG. 18A is a close up partially sectioned perspective view of a lower portion of the traction tower 100', according to an alternative embodiment. As previously discussed with respect to FIG. 1, the upper tower 3' includes an alignment peg 3-3, which is configured to fit into a corresponding elongated hole/slot 2-3 in the lower tower 2. Alignment peg 3-3 helps a user easily position the upper tower 3' onto the lower tower 2 before installing the tower locking knob 4. It also makes sure that the upper tower 3' stays vertical if/when a user is adjusting the height of the upper tower 3', because there are two pins/stems in the slot 2-3 of the lower tower (the pin 3-3 from the upper tower 3' and the threaded rod/stem 4-1 from the tower locking knob 4). This way, if the height of the upper tower 3' is adjusted after the full tower is assembled, there is no risk of the whole upper portion (arm assembly) of the tower 100' rotating and swinging down when the tower locking knob 4 is loosened. In this embodiment, height adjustment can be done with the tower locking knob 4 loosened, not removed entirely from the lower tower/upper tower.

Turning to FIG. 18B-C, a close up transparent perspective view and a solid view, respectively, of a lower portion of the traction tower 100' is shown, according to an alternative embodiment. The lower tower 2 includes a sliding button 2-1, which is attached to locking peg 2-2. Spring 2-4 biases sliding button 2-1 and peg 2-2 in the downward direction, which can be overcome by a user sliding button 2-1 up moving peg 2-2 within the body of lower tower 2. The purpose of peg 2-2 and attachment means 15 (here a key locking feature) is to lock the lower tower 2 into the base plate 1 when it is installed and to make sure it doesn't move until the tower 100' is ready to be disassembled. It also allows the user to quickly assemble and passively lock it in place when assembling the tower 100' (since peg 2-2 is spring biased to the downward position, the slider button 2-1 does not have to be actuated when assembling - lower tower 2 can just be pushed flush to the base 1 and twisted causing peg 2-2 to fall into base hole 1 when it is at the right rotation position). The key locking feature 15 is placed onto base keyhole 1-1 at the same time to assist with the locking of the lower tower 2 to the base 1 after the twisting motion.

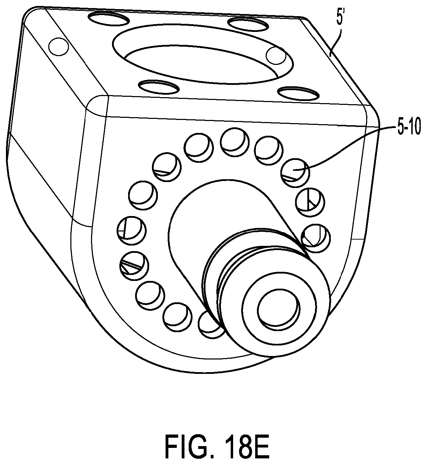

Turning to FIG. 18D, a close up partially sectioned perspective view of a lower portion of the traction tower 100' is shown, according to an alternative embodiment. A slider button 5-7 is shown connected to a locking peg 5-8, which is biased up via spring 5-6 into a hole 5-10 formed into the body of rotation joint 5. To release and freely rotate rotation joint around rotation axis A1, a user can push slider button 5-7 in the downward direction to remove the locking peg from the hole 5-10 until the desired rotation position is reached. Then the button can be released, and the spring 5-6 can move the locking peg 5-8 into another hole 5-10 (see FIG. 18E).

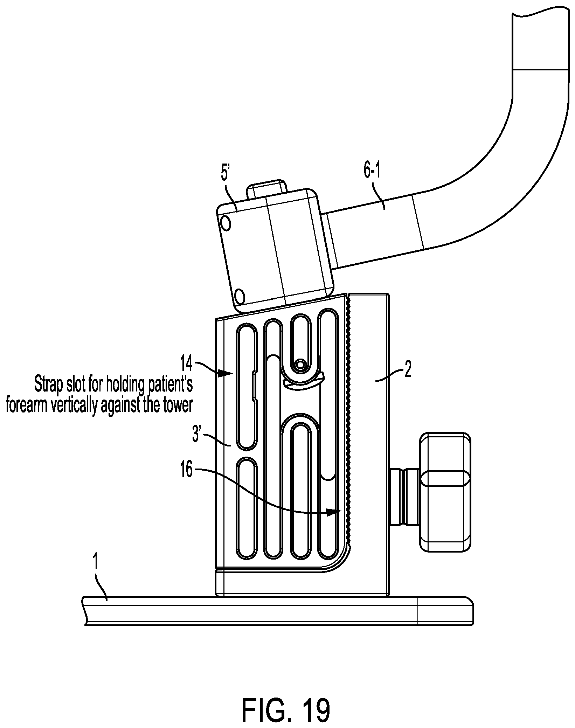

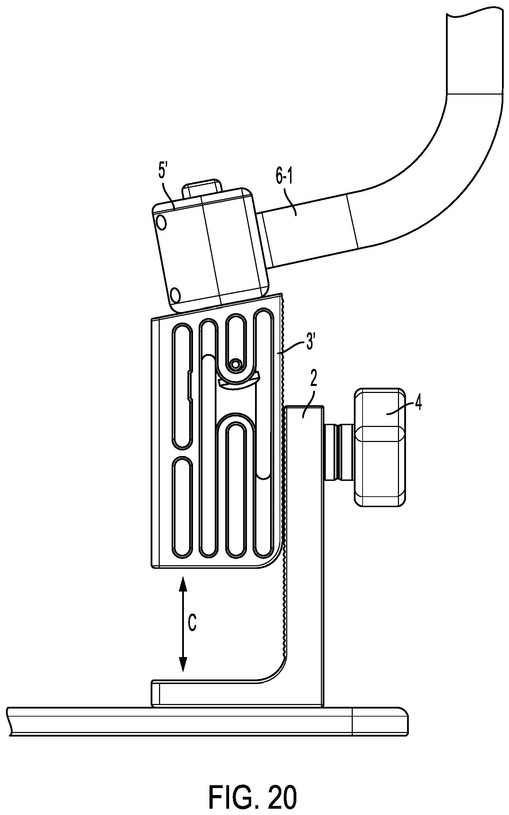

Turning to FIGS. 19 and 20, close up perspective view schematic representations of a lower portion of the traction tower 100' shown in FIG. 15 are provided, according to an alternative embodiment. FIGS. 19 and 20 are similar to FIGS. 4-5, except for the structural differences noted with respect to the discussion herein and above regarding rotation joint 5' and upper tower 3'. However, the same movement of upper tower 3 with respect to lower tower 2 to accommodate a variety of individual patent forearm sizes, and the structural features that allow for such movement, are present in upper tower 3'. FIG. 19 shows the upper tower 3' in its relatively lowest position with respect to lower tower 2, and FIG. 20 shows the upper tower 3' in its relatively highest position with respect to the lower tower 2. The apertures shown other than aperture 14 show where metal has been removed for weight reduction and heat management purposes.

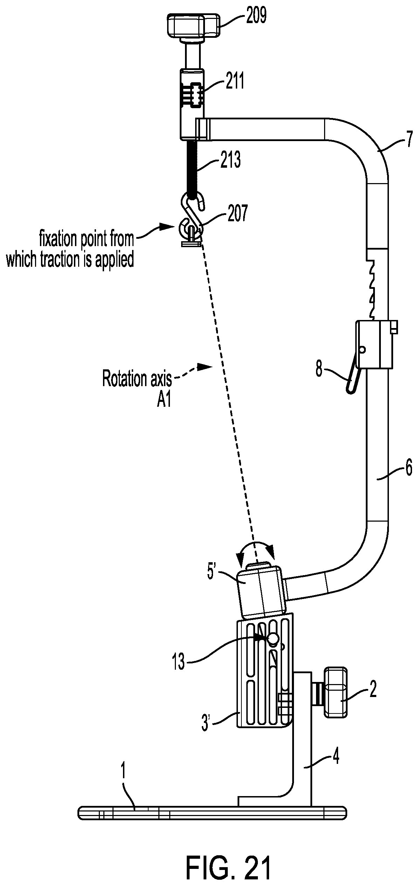

Referring to FIG. 21, a perspective view schematic representation of the traction tower 100' shown in FIG. 15 is provided, according to an alternative embodiment. Similar to FIG. 6, FIG. 21 shows the rotation axis A1 created by angling the rotation joint 5' positioned on and its stem 13 positioned within upper tower 3'.

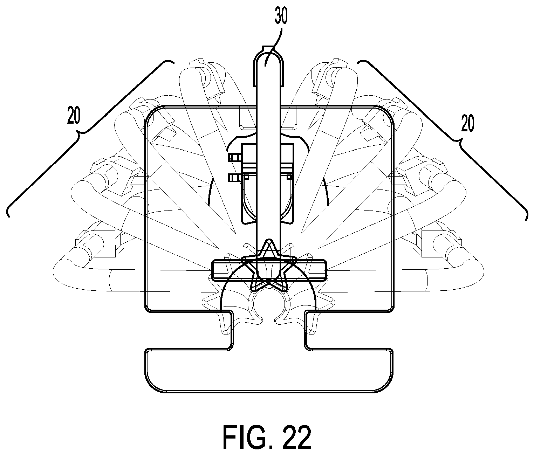

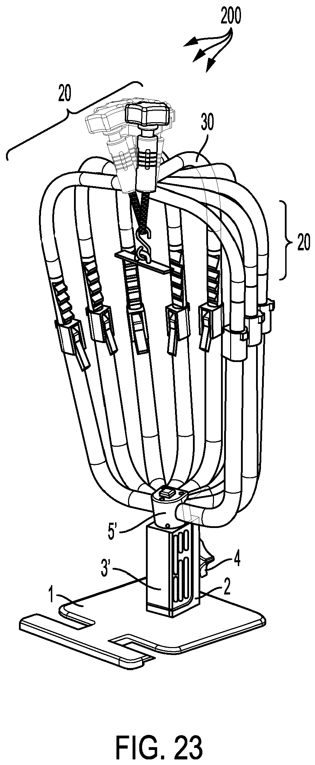

Turning to FIGS. 22 and 23, perspective and top view schematic representations showing the rotation range of rotation joint 5 (and, thus, the arm assembly) of the traction tower 100' about rotation axis A1 are provided. FIGS. 22 and 23 are similar to FIGS. 22 and 23, respectively.

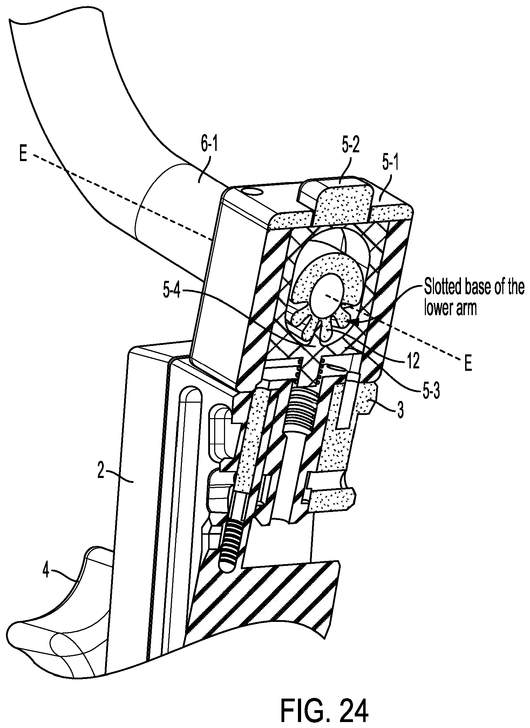

Referring to FIG. 24, a close up partially sectioned perspective view of a lower portion of the traction tower 100' is shown, according to an embodiment. FIG. 24 is similar to FIG. 9, and the elements function in a similar manner even though there are some structural differences with respect to rotation joint 5' and upper tower 3', discussed above.

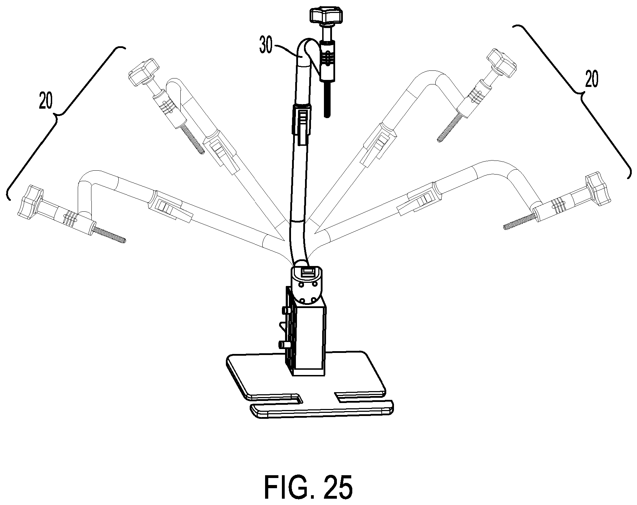

Referring to FIG. 25, a perspective view schematic representation showing the rotation range of the elongated lower end 6-1 of the lower arm 6 (and, thus, the arm assembly) of the traction tower 100 about rotation axis E---E. FIG. 25 is similar to FIGS. 10 and 11.

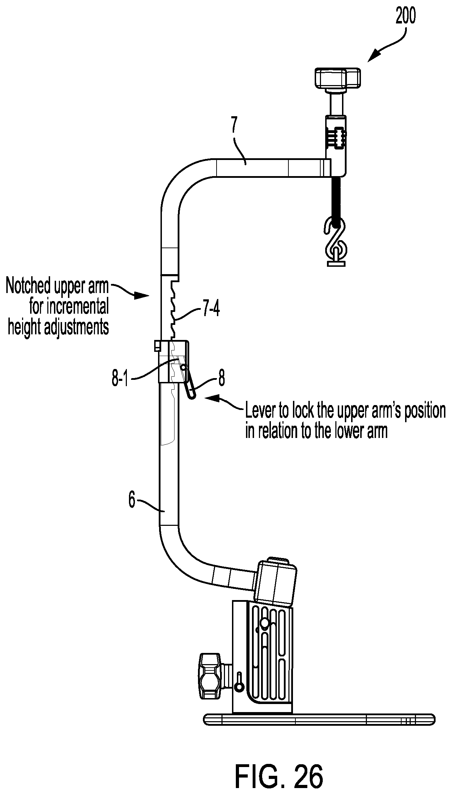

Turning to FIG. 26, a perspective view schematic representation of the traction tower 100' is shown with a height adjustment mechanism including the lever 8 and ridges 7-4 engagement structure and resulting functionality in a partial transparent view, according to an alternative embodiment. FIG. 26 is similar to FIG. 12.

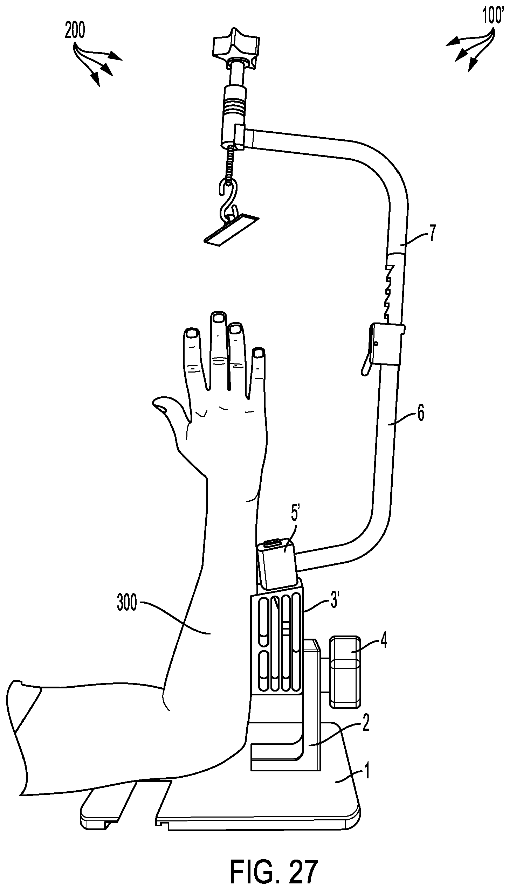

Turning to FIG. 27, a perspective view photographic representation of the traction tower 100' is shown, according to an alternative embodiment. FIG. 27 shows the placement of a patient's arm with respect to the traction tower assembly 100'.

It should be understood that the values used above are only representative values, and other values may be in keeping within the scope of the invention.

While several inventive embodiments have been described and illustrated herein with reference to certain exemplary embodiments, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein (and it will be understood by one skilled in the art that various changes in detail may be effected therein without departing from the scope of the invention as defined by claims that can be supported by the written description and drawings). More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto; inventive embodiments may be practiced otherwise than as specifically described and claimed. Further, where exemplary embodiments are described with reference to a certain number of elements it will be understood that the exemplary embodiments can be practiced utilizing either less than or more than the certain number of elements.

All definitions, as defined and used herein, should be understood to control over dictionary definitions, definitions in documents mention above and/or ordinary meanings of the defined terms.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if not directly attached to where there is something intervening.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

Approximating language, as used herein throughout the specification and claims, may be applied to modify any quantitative representation that could permissibly vary without resulting in a change in the basic function to which it is related. Accordingly, a value modified by a term or terms, such as "about" and "substantially", are not to be limited to the precise value specified. In at least some instances, the approximating language may correspond to the precision of an instrument for measuring the value. Here and throughout the specification and claims, range limitations may be combined and/or interchanged; such ranges are identified and include all the sub-ranges contained therein unless context or language indicates otherwise.

The recitation of ranges of values herein are merely intended to serve as a shorthand method of referring individually to each separate value falling within the range, unless otherwise indicated herein, and each separate value is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate embodiments of the invention and does not impose a limitation on the scope of the invention unless otherwise claimed.

No language in the specification should be construed as indicating any non-claimed element as essential to the practice of the invention.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

It will be apparent to those skilled in the art that various modifications and variations can be made to the present invention without departing from the scope of the invention. Thus, it is intended that the present invention cover the modifications and variations of this invention provided they come within the scope of the appended claims.

## Claims

1. A traction tower assembly for facilitating arthroscopic surgical procedures, radiographic procedures and other related medical procedures on a patient's wrist, comprising:
a base plate (1) for supporting the tower assembly on a stand surface;
a first tower component (2) removably locked to the base plate;
a second tower component (3) connected to the first tower component for linear displacement along a first axis (B-B), with a locking mechanism for selectively fixing the second tower component against displacement at the first tower component;
an elongated arm assembly (6, 7) attached to and extending from the second tower component, the arm assembly having a distal terminal end (7-3) for supporting a patient's hand at the traction tower assembly; and
a rotation joint (5) interfacing a proximal terminal end of the elongate arm assembly with the second tower component, the rotation joint configured to allow both (i) rotation of the elongate arm assembly about a second axis (A1) inclined with respect to the first axis and (ii) rotation of the elongate arm about a third axis (E-E) inclined with respect to the plane of the base plate.

2. The traction tower assembly of claim 1, wherein the plane of the base plate (1) locates in a horizontal plane, wherein the first axis (B-B) is substantially vertical, and wherein an inclination angle of the second axis (A1) with respect to the first axis is the same as an inclination angle of the third axis (E-E) with respect to the horizontal plane.

3. The traction tower assembly of claim 1 or 2, wherein second axis (A1) and the third axis (E-E) extend perpendicular to one another.

4. The traction tower assembly of anyone of claim 1 to 3, wherein the rotation joint (5) comprises a head (5-1) having a bottom surface, a top surface and a side surface, wherein the bottom surface is positioned on a top surface (3-1) of the second tower component (3) that extends parallel to the bottom surface, and wherein the proximal terminal end (12) of the elongate arm assembly (6, 7) is positioned through the side surface of the head.

5. The traction tower assembly of claim 4, wherein the second axis (A1) is positioned through a center of the top surface of the head (5-1) and through a center of the bottom surface of the head, and wherein the head of the rotation joint (5) and the proximal terminal end (12) of the elongate arm assembly (6, 7) are configured to jointly rotate about the second axis with respect to the second tower component (3).

6. The traction tower assembly of claim 5, wherein the bottom surface of the head (5-1) extends in a plane parallel to the third axis (E-E) and inclined with respect to the horizontal plane.

7. The traction tower of any one of claims 4 to 6, wherein the rotation joint (5) comprises a stem (13) protruding from the bottom surface of the head and rotatably received in the second tower component (3), the second axis (A1) positioned through a center of the stem.

8. The traction tower of claim 7, further comprising a locking mechanism (5-2, 5-3, 5-4) for selectively locking / unlocking the stem (13) against rotation with respect to the second tower component (3).

9. The traction tower assembly of any one of claims 4 to 8, wherein the third axis (B-B) is positioned through a center of the side surface of the head (5-1) and through a center of the proximal terminal end (12) of the elongate arm assembly (6, 7).

10. The traction tower assembly of claim 7, wherein the proximal terminal end (12) of the elongate arm assembly (6, 7) is configured for stepwise rotation about the third axis (E-E) with respect to the head (5-1) and for being selectively locked in the head against rotation.

11. The traction tower assembly of claim 8, wherein the proximal terminal end (12) of the elongate arm assembly (6, 7) comprises a slotted base end (12) defining a plurality of radial teeth located about the third axis (E-E), and wherein the head (5-1) comprises a locking button (5-2) displaceable against a biasing spring force, the locking button having a locking tooth (5-4) spring-biased to engage between adjoining ones of the radial teeth of the slotted base to lock the elongate arm assembly against rotation.

12. The traction tower assembly of any one of the preceding claims, wherein the elongated arm assembly comprises a first angled arm (6) with a shorter arm portion (6-1) comprising the proximal terminal end (12) and with a longer arm portion (6-2) angled with respect to the shorter arm portion such as to extend parallel to the first axis (A1) in upward direction from the traction tower assembly.

13. The traction tower assembly of claim 12, wherein the elongated arm assembly further comprises a second angled arm (7) having a proximal arm portion (7-1) extending co-axially with and connected in axially but not rotationally displaceable manner to the longer arm portion (6-2) of the first angled arm (6), and a distal arm portion (7-3) angled 90° with respect to the proximal arm portion.

14. The traction tower assembly of claim 13, further comprising an actuatable locking mechanism (8) configured to releasably lock the second angled arm (7) in place with respect to the first angled arm (6).

15. The traction tower assembly of claim 13 or 14, further comprising a traction scale (200) attached near or at a terminal end (7-5) of the distal arm portion (7-3) of the second angled arm (7), wherein the traction scale is configured to be coupled to a patient's hand and indicate a relative traction amount applied to the patient's arm/wrist when in use, and wherein a geometry of the first and second angled arms (6, 7) and the angle of the second axis (A1) of rotation vis the first axis (B--B) are selected such that a point where the traction scale attaches to the second angled arm remains substantially fixed in space for different rotational positions of the elongate arm assembly in relation to the second tower component (3) and about the second axis, this structural configuration and associated functionality allowing a medical practitioner to move the elongate arm assembly around the patient's hand without affecting the position or traction of the hand itself.

## Patentansprüche

1. Traktionsturmanordnung zum Ermöglichen arthroskopischer chirurgischer Eingriffe, radiographischer Verfahren und anderer damit verbundener medizinischer Verfahren am Handgelenk eines Patienten, umfassend:
eine Basisplatte (1) zum Halten der Turmanordnung auf einer Standfläche;
eine erste Turmkomponente (2), die lösbar an der Basisplatte verriegelt ist;
eine zweite Turmkomponente (3), die mit der ersten Turmkomponente zur linearen Verschiebung entlang einer ersten Achse (B-B) verbunden ist, mit einem Verriegelungsmechanismus zur selektiven Fixierung der zweiten Turmkomponente gegen Verschiebung an der ersten Turmkomponente;
eine verlängerte Armanordnung (6, 7), die an der zweiten Turmkomponente angebracht ist und von dieser ausgeht, wobei die Armanordnung ein distales Endstück (7-3) zum Halten einer Patientenhand an der Traktionsturmanordnung aufweist; und
ein Drehgelenk (5), das ein proximales Endstück der verlängerten Armanordnung mit der zweiten Turmkomponente koppelt, wobei das Drehgelenk dazu eingerichtet ist, sowohl (i) eine Drehung der verlängerten Armanordnung um eine zweite Achse (A1), die in Bezug auf die erste Achse geneigt ist, als auch (ii) eine Drehung des verlängerten Arms um eine dritte Achse (E-E), die in Bezug auf die Ebene der Basisplatte geneigt ist, zu ermöglichen.

2. Traktionsturmanordnung nach Anspruch 1, wobei sich die Ebene der Basisplatte (1) in einer horizontalen Ebene befindet, wobei die erste Achse (B-B) im Wesentlichen vertikal ist und wobei ein Neigungswinkel der zweiten Achse (A1) in Bezug auf die erste Achse derselbe ist wie ein Neigungswinkel der dritten Achse (E-E) in Bezug auf die horizontale Ebene.

3. Traktionsturmanordnung nach Anspruch 1 oder 2, wobei die zweite Achse (A1) und die dritte Achse (E-E) senkrecht zueinander verlaufen.

4. Traktionsturmanordnung nach einem der Ansprüche 1 bis 3, wobei das Drehgelenk (5) einen Kopf (5-1) mit einer unteren Oberfläche, einer oberen Oberfläche und einer seitlichen Oberfläche umfasst, wobei die untere Oberfläche auf einer oberen Oberfläche (3-1) der zweiten Turmkomponente (3) positioniert ist, die parallel zur unteren Oberfläche verläuft, und wobei das proximale Endstück (12) der verlängerten Armanordnung (6, 7) durch die seitliche Oberfläche des Kopfes positioniert ist.

5. Traktionsturmanordnung nach Anspruch 4, wobei die zweite Achse (A1) durch eine Mitte der oberen Oberfläche des Kopfes (5-1) und durch eine Mitte der unteren Oberfläche des Kopfes positioniert ist, und wobei der Kopf des Drehgelenks (5) und das proximale Endstück (12) der verlängerten Armvorrichtung (6, 7) dazu eingerichtet sind, sich gemeinsam um die zweite Achse in Bezug auf die zweite Turmkomponente (3) zu drehen.

6. Traktionsturmanordnung nach Anspruch 5, wobei die untere Oberfläche des Kopfes (5-1) in einer Ebene parallel zur dritten Achse (E-E) und geneigt in Bezug auf die horizontale Ebene verläuft.

7. Traktionsturm nach einem der Ansprüche 4 bis 6, wobei das Drehgelenk (5) einen Schaft (13) umfasst, der von der unteren Oberfläche des Kopfes vorsteht und drehbar in der zweiten Turmkomponente (3) aufgenommen ist, wobei die zweite Achse (A1) durch eine Mitte des Schafts positioniert ist.

8. Traktionsturm nach Anspruch 7, ferner umfassend einen Verriegelungsmechanismus (5-2, 5-3, 5-4) zur selektiven Verriegelung/Entriegelung des Schafts (13) gegen Drehung in Bezug auf die zweite Turmkomponente (3).

9. Traktionsturmanordnung nach einem der Ansprüche 4 bis 8, wobei die dritte Achse (B-B) durch eine Mitte der seitlichen Oberfläche des Kopfes (5-1) und durch eine Mitte des proximalen Endstücks (12) der verlängerten Armanordnung (6, 7) positioniert ist.

10. Traktionsturmanordnung nach Anspruch 7, wobei das proximale Endstück (12) der verlängerten Armanordnung (6, 7) zu einer schrittweisen Drehung um die dritte Achse (E-E) in Bezug auf den Kopf (5-1) und zu einer selektiven Verriegelung im Kopf gegen Drehung eingerichtet ist.

11. Traktionsturmanordnung nach Anspruch 8, wobei das proximale Endstück (12) der verlängerten Armanordnung (6, 7) ein geschlitztes Basisende (12) umfasst, das mehrere radiale Zähne definiert, die um die dritte Achse (E-E) angeordnet sind, und wobei der Kopf (5-1) eine Verriegelungstaste (5-2) umfasst, die gegen eine vorgespannte Federkraft verschiebbar ist, wobei die Verriegelungstaste einen Verriegelungszahn (5-4) aufweist, der federvorgespannt ist, um zwischen benachbarten der radialen Zähne der geschlitzten Basis einzugreifen, um die verlängerte Armanordnung gegen Drehung zu verriegeln.

12. Traktionsturmanordnung nach einem der vorhergehenden Ansprüche, wobei die verlängerte Armanordnung einen ersten abgewinkelten Arm (6) mit einem kürzeren Armabschnitt (6-1), der das proximale Endstück (12) umfasst, und mit einem längeren Armabschnitt (6-2) umfasst, der in Bezug auf den kürzeren Armabschnitt abgewinkelt ist, um so parallel zur ersten Achse (A1) in Aufwärtsrichtung von der Traktionsturmanordnung aus zu verlaufen.

13. Traktionsturmanordnung nach Anspruch 12, wobei die verlängerte Armanordnung ferner einen zweiten abgewinkelten Arm (7), der einen proximalen Armabschnitt (7-1) aufweist, der koaxial mit dem längeren Armabschnitt (6-2) des ersten abgewinkelten Arms (6) verläuft und in axial aber nicht rotierend verschiebbarer Weise mit diesem verbunden ist, und einen distalen Armabschnitt (7-3), der um 90° in Bezug auf den proximalen Armabschnitt abgewinkelt ist, umfasst.

14. Traktionsturmanordnung nach Anspruch 13, ferner umfassend einen betätigbaren Verriegelungsmechanismus (8), der dazu eingerichtet ist, den zweiten abgewinkelten Arm (7) lösbar in Position in Bezug auf den ersten abgewinkelten Arm (6) zu verriegeln.

15. Traktionsturmanordnung nach Anspruch 13 oder 14, ferner umfassend eine Traktionswaage (200), die nahe oder am Endstück (7-5) des distalen Armabschnitts (7-3) des zweiten abgewinkelten Arms (7) angebracht ist, wobei die Traktionswaage dazu eingerichtet ist, mit der Hand eines Patienten gekoppelt zu werden und eine relatives Maß an Traktion anzuzeigen, die auf den Arm/das Handgelenk des Patienten bei Verwendung angelegt wird, und wobei eine Geometrie des ersten und des zweiten abgewinkelten Arms (6, 7) und der Winkel der zweiten Drehachse (A1) gegenüber der ersten Achse (B-B) so gewählt sind, dass ein Punkt, an dem die Traktionswaage am zweiten abgewinkelten Arm angebracht ist, für verschiedene Rotationspositionen der verlängerten Armanordnung in Bezug auf die zweite Turmkomponente (3) und um die zweite Achse im Wesentlichen fest im Raum bleibt, wobei diese strukturelle Einrichtung und die damit verbundene Funktionalität es einem Mediziner ermöglichen, die verlängerte Armanordnung um die Hand des Patienten zu bewegen, ohne die Position oder Traktion der Hand selbst zu beeinflussen.

## Revendications

1. Ensemble de tour de traction pour faciliter des procédures chirurgicales arthroscopiques, des procédures radiographiques et autres procédures médicales associées sur un poignet d'un patient, comprenant :
une plaque (1) de base pour supporter l'ensemble de tour sur une surface de socle ;
un premier composant (2) de tour verrouillé de façon amovible sur la plaque de base ;
un deuxième composant (3) de tour connecté au premier composant de tour pour un déplacement linéaire le long d'un premier axe (B-B) avec un mécanisme de verrouillage pour sélectivement fixer le deuxième composant de tour contre un déplacement au niveau du premier composant de tour ;
un ensemble (6, 7) de bras allongé fixé au et s'étendant depuis le deuxième composant de tour, l'ensemble de bras ayant une extrémité terminale distale (7-3) pour supporter une main d'un patient sur l'ensemble de tour de traction ; et
une articulation rotative (5) faisant interface entre une extrémité terminale proximale de l'ensemble de bras allongé et le deuxième composant de tour, l'articulation rotative configurée pour permettre à la fois (i) une rotation de l'ensemble de bras allongé autour d'un deuxième axe (A1) incliné par rapport au premier axe et (ii) une rotation du bras allongé autour d'un troisième axe (E-E) incliné par rapport au plan de la plaque de base.

2. Ensemble de tour de traction selon la revendication 1, dans lequel le plan de la plaque (1) de base se situe dans un plan horizontal, dans lequel le premier axe (B-B) est sensiblement vertical, et dans lequel un angle d'inclinaison du deuxième axe (A1) par rapport au premier axe est le même qu'un angle d'inclinaison du troisième axe (E-E) par rapport au plan horizontal.

3. Ensemble de tour de traction selon la revendication 1 ou 2, dans lequel le deuxième axe (A1) et le troisième axe (E-E) s'étendent perpendiculaires l'un à l'autre.

4. Ensemble de tour de traction selon l'une quelconque des revendications 1 à 3, dans lequel l'articulation rotative (5) comprend une tête (5-1) ayant une surface inférieure, une surface supérieure et une surface latérale, dans lequel la surface inférieure est positionnée sur une surface supérieure (3-1) du deuxième composant (3) de tour qui s'étend parallèle à la surface inférieure, et dans lequel l'extrémité terminale proximale (12) de l'ensemble (6, 7) de bras allongé est positionnée au travers de la surface latérale de la tête.

5. Ensemble de tour de traction selon la revendication 4, dans lequel le deuxième axe (A1) est positionné au travers d'un centre de la surface supérieure de la tête (5-1) et au travers d'un centre de la surface inférieure de la tête, et dans lequel la tête de l'articulation rotative (5) et l'extrémité terminale proximale (12) de l'ensemble (6, 7) de bras allongé sont configurées pour tourner conjointement autour du deuxième axe par rapport au deuxième composant (3) de tour.

6. Ensemble de tour de traction selon la revendication 5, dans lequel la surface inférieure de la tête (5-1) s'étend dans un plan parallèle au troisième axe (E-E) et incliné par rapport au plan horizontal.

7. Ensemble de tour de traction selon l'une quelconque des revendications 4 à 6, dans lequel l'articulation rotative (5) comprend une tige (13) faisant saillie depuis la surface inférieure de la tête et reçue rotative dans le deuxième composant (3) de tour, le deuxième axe (A-1) positionné au travers d'un centre de la tige.

8. Ensemble de tour de traction selon la revendication 7, comprenant en outre un mécanisme (5-2, 5-3, 5-4) de verrouillage pour sélectivement verrouiller/déverrouiller la tige (13) contre une rotation par rapport au deuxième composant (3) de tour.

9. Ensemble de tour de traction selon l'une quelconque des revendications 4 à 8, dans lequel le troisième axe (E-E) est positionné au travers d'un centre de la surface latérale de la tête (5-1) et au travers d'un centre de l'extrémité terminale proximale (12) de l'ensemble (6, 7) de bras allongé.

10. Ensemble de tour de traction selon la revendication 7, dans lequel l'extrémité terminale proximale (12) de l'ensemble (6, 7) de bras allongé est configurée pour une rotation pas à pas autour du troisième axe (E-E) par rapport à la tête (5-1) et pour être sélectivement verrouillée dans la tête contre une rotation.

11. Ensemble de tour de traction selon la revendication 8, dans lequel l'extrémité terminale proximale (12) de l'ensemble (6, 7) de bras allongé comprend une extrémité de base (12) fendue définissant une pluralité de dents radiales situées autour du troisième axe (E-E), et dans lequel la tête (5-1) comprend un bouton (5-2) de verrouillage déplaçable contre une force de ressort de poussée, le bouton de verrouillage ayant une dent (5-4) de verrouillage poussée par ressort pour un engagement entre celles adjacentes des dents radiales de la base fendue pour verrouiller l'ensemble de bras allongé contre une rotation.

12. Ensemble de tour de traction selon l'une quelconque des revendications précédentes, dans lequel l'ensemble de bras allongé comprend un premier bras en angle (6) avec une partie de bras (6-1) plus courte comprenant l'extrémité terminale proximale (12) et avec une partie de bras (6-2) plus longue en angle par rapport à la partie de bras plus courte de façon à s'étendre parallèle au premier axe (A1) dans un sens vers le haut depuis l'ensemble de tour de traction.

13. Ensemble de tour de traction selon la revendication 12, dans lequel l'ensemble de bras allongé comprend en outre un deuxième bras en angle (7) ayant une partie proximale (7-1) de bras s'étendant coaxialement avec et connecté déplaçable d'une façon axiale mais non rotative à la partie de bras (6-2) plus longue du premier bras en angle (6), et une partie distale (7-3) de bras en angle de 90° par rapport à la partie proximale de bras.

14. Ensemble de tour de traction selon la revendication 13, comprenant en outre un mécanisme (8) de verrouillage actionnable configuré pour verrouiller de façon libérable le deuxième bras en angle (7) en place par rapport au premier bras en angle (6).

15. Ensemble de tour de traction selon la revendication 13 ou 14, comprenant en outre une échelle (200) de traction fixée à proximité ou au niveau d'une extrémité terminale (7-5) de la partie distale (7-3) de bras du deuxième bras en angle (7), dans lequel l'échelle de traction est configurée pour être couplée à une main d'un patient et indiquer une quantité de traction relative appliquée à un bras/poignet du patient lors de l'utilisation, et dans lequel une géométrie des premier et deuxième bras en angle (6, 7) et l'angle du deuxième axe (A1) de rotation par rapport au premier axe (B-B) sont sélectionnés de telle sorte qu'un point où l'échelle de traction se fixe au deuxième bras en angle reste sensiblement fixe dans l'espace pour différentes positions en rotation de l'ensemble de bras allongé en relation au deuxième composant (3) de tour et autour du deuxième axe, cette configuration structurelle et cette fonctionnalité associée permettant à un praticien médical de déplacer l'ensemble de bras allongé autour de la main du patient sans affecter la position ou la traction de la main elle-même.
